# EUROPÄISCHE PATENTSCHRIFT

(11) **EP 1 805 151 B1**
(45) Veröffentlichungstag und Bekanntmachung des Hinweises auf die Patenterteilung: **19.12.2007**
(21) Anmeldenummer: 05799775.1
(22) Anmeldetag: 19.10.2005
(51) Int. Cl.: C07D 251/60

(54) **VERFAHREN ZUR BEHANDLUNG VON TRIAZINHALTIGEM WASSER EINER MELAMINANLAGE**
METHOD FOR THE TREATMENT OF TRIAZINE-CONTAINING WATER OF A MELAMINE PLANT
PROCEDE DE TRAITEMENT D'UNE EAU CONTENANT DES TRIAZINES DANS UNE USINE DE MELAMINE

(30) Priorität: 20.10.2004 DE 102004051432
(43) Veröffentlichungstag der Anmeldung: 11.07.2007
(73) Patentinhaber: AMI Agrolinz Melamine International GmbH, 4021 Linz (AT)
(72) Erfinder: TOPLACK, Paul, A-8010 Graz (AT); WEISS, Peter, A-4061 Pasching (AT); BUCKA, Hartmut, A-4622 Eggendorf (AT)
(74) Vertreter: Gross, Felix
(86) Internationale Anmeldenummer: PCT/EP2005/011373
(87) Internationale Veröffentlichungsnummer: WO 2006/042760

(56) Entgegenhaltungen:
- WO-A-00/71525
- WO-A-01/46159
- WO-A-02/100839
- DE-A1- 1 930 844
- US-A- 3 423 411

## Beschreibung

Die Erfindung betrifft ein Verfahren zur Behandlung von triazinhaltigem Wasser einer Melaminanlage sowie ein Hochdruckverfahren zur Herstellung von Melamin aus Harnstoff.

Als Ausgangsprodukt für die Melaminherstellung dient nahezu ausschließlich Harnstoff, welcher entweder in einem katalytischen Niederdruckverfahren oder in einem Hochdruckverfahren ohne Katalysator zu Melamin umgesetzt wird.

Die im Rohmelamin vorhandenen Nebenprodukte, Abbauprodukte und nichtumgesetzte Ausgangsstoffe müssen bei der anschließenden Melaminaufarbeitung vom Produkt abgetrennt werden. Da die Melaminaufarbeitung meist in Gegenwart von Wasser erfolgt, werden diese Nebenprodukte als Abwasserinhaltsstoffe, beispielsweise als Inhaltsstoffe der Mutterlauge aus der Melaminkristallisation, erhalten.

Die Abwasserinhaltsstoffe aus dem Melaminprozess sind hauptsächlich zyklische Stickstoffverbindungen in Form von Triazinen wie Melamin oder Ureidomelamin oder Oxoaminotriazine (OAT), wie Ammelin oder Ammelid. Darüber hinaus können auch Harnstoff oder Cyanursäure enthalten sein. Aufgrund der wässrigen Melaminaufarbeitung im basischen Milieu liegen diese Stoffe großteils ionisch vor. Als weitere ionische Inhaltsstoffe des Melaminabwassers können beispielsweise Carbonate, Natrium- oder Ammoniumionen vorkommen.

Im Sinne einer möglichst hohen Melaminausbeute ist es gewünscht, das im Wasser enthaltene Melamin möglichst vollständig und selektiv rückzugewinnen und in den Melaminprozess zu rezyklieren. Dies bedingt eine Trennung des Melamins von den anderen Abwasserinhaltsstoffen.

Aus dem Stand der Technik sind einige Verfahren zur Behandlung von triazinhaltigen Melaminabwässern bekannt, welche das Melamin von den OAT separieren. Bei einigen dieser Verfahren werden die OAT als Feststoffe rückgewonnen.

Gemäß WO 01/46159 A2 beispielsweise wird die Melamin und OAT enthaltende Mutterlauge nach der Melaminkristallisation bis pH = 7 angesäuert, wodurch eine OAT Suspension entsteht, welche anschließend einer Tangentialfiltration unterzogen wird. Dadurch werden eine wässrige Melaminlösung und eine OAT Dispersion erhalten. Während die wässrige Melaminlösung in den Prozess rezykliert wird, werden aus der Dispersion die OAT separiert. Nachteilig bei diesem Verfahren ist unter anderem, dass ein Feststoffe enthaltender Strom filtriert wird; dadurch besteht die Gefahr einer Filterverstopfung.

Gemäß IT 0 128 2369 wird das Melamin und OAT enthaltende Abwasser bei hoher Temperatur und hohem Druck behandelt, was eine Zerstörung der OAT bewirkt. Nachdem das dabei entstehende NH₃ und CO₂ abgestrippt wird, wird die zurückbleibende Lösung in die Melaminanlage rückgeführt. Bei diesem Verfahren besteht der Nachteil, dass bei der thermischen Behandlung nicht nur die OAT sondern auch das im Abwasser enthaltene Melamin zerstört wird und demnach keine befriedigende Melaminausbeute im Gesamtprozess erzielt werden kann.

In WO 02/100839 A1 wird ein Verfahren beschrieben, bei welchem ein Großteil der Mutterlaugen der Melaminkristallisation unbehandelt in die Melaminanlage rückgeführt wird; aus dem kleineren Teil der Mutterlaugen werden NH₃ und OAT rückgewonnen. Der Nachteil dieses Verfahrens ist der, dass bei der Rückführung der unbehandelten, OAT-haltigen Mutterlaugen in den Melaminprozess mehr Frischwasser zusätzlich zugeführt werden muss, um die Rohmelaminschmelze bis zur gewünschten Qualität aufarbeiten zu können.

Es stellte sich demnach die Aufgabe, ein Verfahren für die Abwasserbehandlung zu entwickeln, bei dem in flüssiger Phase gearbeitet werden kann und bei dem eine gute Separation zwischen Melamin und sonstigen Abwasserinhaltsstoffen erreicht wird, so dass möglichst viel Melamin in den Prozess rückgeführt werden kann.

Gegenstand der vorliegenden Erfindung ist demnach ein Verfahren zur Behandlung von triazinhaltigem Wasser einer Melaminanlage, das dadurch gekennzeichnet ist, dass
- das ionische und nichtionische Triazine in gelöster Form enthaltende Wasser mindestens einer Membranfiltrationseinheit zugeführt wird,
- das Wasser in der Membranfiltrationseinheit in eine an ionischen Triazinen reiche und eine an nichtionischen Triazinen reiche Fraktion aufgetrennt wird,
- worauf die an ionischen Triazinen reiche Fraktion ausgeschleust und die an nichtionischen Triazinen reiche Fraktion in die Melaminanlage rückgeführt wird.

Das im erfindungsgemäßen Verfahren behandelte Wasser kann aus jeder beliebigen Melaminanlage stammen.

Das Wasser enthält sowohl ionische als auch nichtionische Triazine und weist im allgemeinen einen basischen pH Wert auf.

Zu den ionischen Triazinen gehören beispielsweise die Salze der OAT, im allgemeinen die Natrium -Salze von Ammelin, Ammelid und Cyanursäure. Darüber hinaus können im Wasser weitere ionische Verbindungen wie beispielsweise Carbonate und/oder Kationen wie Natrium oder Ammonium enthalten sein.

Als nichtionische Triazine sind beispielsweise Melamin, Ureidomelamin oder Cyanursäure im Wasser enthalten. Andere nichtionische Inhaltsstoffe wie beispielsweise Harnstoff oder weitere Stickstoffverbindungen können ebenfalls enthalten sein.

Entscheidend ist, dass die Stoffe im Wasser großteils in gelöster Form vorliegen. Bei Vorliegen von zu vielen festen Bestandteilen besteht die Gefahr der Membranverstopfung. Da die Löslichkeit der Inhaltsstoffe vom pH Wert abhängig ist, kann gegebenenfalls vor der Zufuhr des Wassers zur Membranfiltrationseinheit der pH Wert durch Zugabe von Lauge bis zum Erreichen vollständiger Löslichkeit erhöht werden.

Vorteilhafterweise besteht die Membranfiltrationseinheit aus einem Membranmodul, einem Druckrohr, einer Hochdruckpumpe, einem Vorlage- und Sammelbehälter und einer Zirkulationspumpe, wobei die Membranmodule vorteilhafterweise als Wickelmodule ausgeführt sind.

Die Membranfiltrationseinheit kann eine oder mehrere Filtrationsstufen umfassen. Bei einer mehrstufigen Membranfiltrationseinheit wird das Permeat der ersten Membranfiltrationseinheit mindestens einer weiteren Membranfiltrationseinheit zugeführt. Bei einer mehrstufigen Filtration werden höhere Reinheiten des Permeates erreicht. Es ist in einer mehrstufigen Membranfiltrationseinheit auch möglich, Membranmodule mit unterschiedlichen Trenneigenschaften zu verwenden. Auf diese Weise können je nach Zusammensetzung des triazinhaltigen Wassers optimale Trenngrade erreicht werden. Die Retentate der einzelnen Membranfiltrationseinheiten können vereinigt oder jedes für sich seiner weiteren Verwendung zugeführt werden.

In der Membranfiltrationseinheit wird das Wasser in zwei Fraktionen aufgetrennt. Es handelt sich bei der Membranfiltration vorteilhafterweise um eine Druckfiltration, wobei die Auftrennung der Fraktionen über die ionischen Eigenschaften der Inhaltsstoffe erfolgt. Je höher der angewandte Druck ist, desto höher ist der Durchsatz über die Membranfiltrationseinheit.

Nach der Membranfiltrationseinheit wird die an ionischen Triazinen reiche Fraktion, die einen Großteil der unerwünschten OAT enthält, ausgeschleust. Beispielsweise wird sie einer thermischen Abwasserbehandlungsanlage, in welcher die OAT abgebaut werden, zugeführt.

Die an nichtionischen Triazinen, insbesondere an Melamin reiche Fraktion nach der Membranfiltrationseinheit kann in eine beliebige Verfahrensstufe der Melaminanlage rezykliert werden. Sie wird beispielsweise in den Quencher, in den Verwellzeitreaktor oder in den Kristallisator des Nassteils einer Hochdruckmelaminanlage rückgeführt. Im Falle einer mehrstufigen Membranfiltrationsanlage ist es auch möglich, zumindest einen Teil des nach den ersten Filtrationsstufen teilgereinigten Wassers bereits in die Melaminanlage rückzuführen. Es kann dort beispielsweise als Waschwasser Anwendung finden. Durch die Rückführung des melaminhaltigen Wassers wird die Melaminausbeute des Gesamtprozesses erhöht.

Bevorzugt enthält das triazinhaltige Wasser die bei der Melaminkristallisation aus wässriger Lösung anfallende Mutterlauge und weist einen pH Wert von 11 bis 13 auf. Bei Verwendung der Kristallisationsmutterlauge für das erfindungsgemäße Verfahren ergibt sich die größtmögliche Effizienz, da die Mutterlauge eine besonders hohe Konzentration an ionischen und nichtionischen Triazinen aufweist.

Darüber hinaus kann das triazinhaltige Wasser auch Wasch- oder Spüllösungen aus beliebigen Teilen der Melaminanlage enthalten. Beispielsweise können Waschlösungen aus der der Melaminkristallisation nachgeschalteten Melaminfiltration verwendet werden. Es ist auch möglich, den Kristallisationsmutterlaugenstrom mit Wasch- und/oder Spüllösungen zu mischen, bevor er der Membranfiltrationseinheit zugeführt wird.

Die an ionischen Triazinen, insbesondere Natriumsalze des Ammelids und Ammelins, reiche Fraktion, ist bevorzugt im Retentat enthalten, während die an nichtionischen Triazinen, insbesondere Melamin, reiche Fraktion bevorzugt im Permeat enthalten ist. Dies kann über entsprechende Auswahl des Membrantyps, welcher selektiv gegenüber ionischen Komponenten einen Rückhalt bietet, erreicht werden.

Bevorzugt ist eine Ausführungsform, bei welcher die Membranfiltrationseinheit als Nanofiltration oder Umkehrosmose ausgeführt ist. Sowohl bei der Nanofiltration als auch bei der Umkehrosmose beruht die Trennung auf dem Prinzip der Lösungsdiffusion. Die Nanofiltration erfolgt bei Drücken bis etwa 40 bar. Bei der Umkehrosmose wird von außen ein über dem osmotischen Druck der Lösung liegender Druck bis zu etwa 100 bar aufgewandt, um die Trennung zwischen den ionischen und den nichtionischen Bestandteilen zu erreichen.

Bevorzugt ist eine Ausführungsform, bei welcher die Membran eine aus mehreren Materialschichten aufgebaute Compositmembran ist. Compositmembranen bestehen üblicherweise aus einer Trägermaterialschicht, auf welche eine oder mehrere dünne Membranschichten aufgebracht sind, ein Beispiel dafür sind sogenannte Dünnfilmmembranen. Solche Compositmembranen gewährleisten eine gute mechanische und chemische Stabilität der Membran.

Die Membran kann entweder eine ebene Fläche oder eine gewickelte Fläche sein. Gewickelte Membranflächen sind bevorzugt, da mit ihnen ein hoher Durchsatz bezogen auf das Volumen erreicht werden kann.

Als Membranmaterialien dienen vorzugsweise handelsübliche Polymere wie Polysulfone. Unter den Polysulfonen sind beispielsweise sulfonierte Polysulfone oder Polyethersulfone verwendbar. Auch Polyamidmembranen können eingesetzt werden. Diese Membranmaterialien sind gegenüber den hohen pH-Werten des triazinhaltigen Wassers und den bei der Membranfiltration herrschenden Temperaturen beständig. Für die Materialauswahl der Membran ist neben der chemischen und thermischen Beständigkeit entscheidend, dass das Trennverhalten über die gesamte Betriebsdauer möglichst konstant bleiben soll.

Um eine kontinuierliche Reaktionsführung zu gewährleisten, können zwei oder mehrere Membranfiltrationseinheiten verwendet werden, wobei jeweils eine der Filtrationseinheiten im Reinigungszyklus und die anderen in Betrieb sind. Die Membranreinigung erfolgt beispielsweise durch Spülen mit Heißkondensat oder mit speziellen Reinigungslösungen wie beispielsweise Natronlauge.

Über die Größe der Membranfläche kann der Durchsatz über die Membranfiltrationseinheit gesteuert werden, je größer die Fläche, desto höher ist der Durchsatz.

Bevorzugt ist eine Ausführungsform, bei welcher der Membranfiltrationseinheit eine Ultrafiltration vorgeschaltet ist und das Permeat der Ultrafiltration der Membranfiltrationseinheit zugeführt wird. Die Ultrafiltration dient der Abscheidung von im Wasser enthaltenen Grobbestandteilen oder Schwebstoffen. Auf diese Weise wird eine Schonung und damit eine erhöhte Standzeit der Membran erreicht. Das Trennprinzip bei der Ultrafiltration ähnelt einer mechanischen Filtration, sie erfolgt bei Drücken bis etwa 5 bar.

Die in der Ultrafiltration abgeschiedenen Grobbestandteile sind beispielsweise feste Melaminpartikel, die in die Melaminanlage rückgeführt werden können.

In einer vorteilhaften Ausführungsform wird das triazinhaltige Wasser einer Ultrafiltration zugeführt, das Permeat der Ultrafiltration wird einer Nanofiltration und das Permeat der Nanofiltration einer Umkehrosmose zugeführt. Der Vorteil dieser Ausführungsform liegt darin, dass die Membranfiltrationseinheit durch die vorgeschaltete Ultrafiltration geschützt wird und durch die Serienschaltung zweier Membranfiltrationseinheiten ein hoher Trenngrad bei gleichzeitiger mechanischer Entlastung der einzelnen Filtrationsstufen erreicht wird.

Bevorzugterweise wird die Membranfiltrationseinheit bei einer Temperatur von 20 bis 95 °C, besonders bevorzugt bei 50 bis 70 °C und einem Druck von 0,1 bis 100 bar, besonders bevorzugt bei einem Druck von 10 bis 30 bar betrieben. Diese Parameter sind technisch beherrschbar und liefern ein gutes Trennergebnis.

Bevorzugterweise beträgt das Konzentrationsverhältnis der nichtionischen Triazine im Permeat : Retentat nach der Membranfiltrationseinheit 0,7 bis 1,1. Je höher das Verhältnis ist, umso weniger Melaminverlust kann im Gesamtprozess erreicht werden.
Das Konzentrationsverhältnis der ionischen Komponenten im Permeat : Retentat beträgt vorteilhafterweise 0,0 bis 0,9. Je niedriger das Verhältnis ist, umso weniger Nebenprodukte werden in die Melaminanlage rezykliert.
Das Konzentrationsverhältnis spiegelt den Trenngrad wider. Ein Konzentrationsverhältnis von 1:1 bedeutet, dass die Konzentration einer Komponente im Permeat gleich hoch wie im Retentat ist, der Trenngrad demnach 50 % beträgt.

Über die Auswahl des Membranmaterials und Membranaufbaus kann das Konzentrationsverhältnis beeinflusst werden. Es können auch verschiedene Membrantypen mit unterschiedlichen Trenngraden miteinander kombiniert werden, um für ein gegebenes Trennproblem das optimale Ergebnis zu erzielen.

In einer vorteilhaften Ausführungsform beträgt das Massenverhältnis von Permeat : Retentat 1,5 nach der Membranfiltrationseinheit. Besonders vorteilhaft ist es, wenn das Massenverhältnis von Permeat : Retentat 1,5 bis 20 beträgt. Dadurch wird sichergestellt, dass ein großer Teil des belasteten Wasser nach deren Reinigung in der Membranfiltrationseinheit in den Melaminprozess rückgeführt wird. Dies verursacht eine Einsparung von Frischwasser im Melaminprozess.

Ein weiterer Gegenstand der Erfindung ist ein Hochdruckverfahren zur Herstellung von Melamin aus Harnstoff, das dadurch gekennzeichnet ist, dass nichtionische Triazine, wie Melamin, und ionische Triazine im triazinhaltigen Wasser mindestens einer Membranfiltrationseinheit zugeführt wird und dadurch in eine an ionischen Triazinen reiche und eine an nichtionischen Triazinen reiche Fraktion aufgetrennt wird, worauf die an ionischen Triazinen reiche Fraktion in eine Anlage zur thermischen Abwasserbehandlung bei > 130 °C ausgeschleust und die an nichtionischen Triazinen reiche Fraktion in den Nassteil der Melaminanlage rückgeführt wird.
Dabei ist es möglich, das gesamte triazinhaltige Wasser oder nur einen Teil davon in die Membranfiltrationseinheit zu fahren. Üblicherweise wird ein Teil des triazinhaltigen Wassers ohne Behandlung in der Membranfiltrationseinheit in die Melaminanlage rückgeführt und ein zweiter Teil wird nach der erfindungsgemäßen Behandlung in der Membranfiltrationseinheit rückgeführt.

In der thermischen Abwasserbehandlung findet beispielsweise in einem Hydrolizer unter hohem Druck und hoher Temperatur eine Zersetzung der ionischen Triazine und weiteren Inhaltsstoffe statt. Das dabei entstehende CO₂ und NH₃ kann als Ammoncarbonat wiedergewonnen und an geeigneter Stelle in die Melamin- oder Harnstoffanlage rückgeführt werden, während das gereinigte Wasser zur Entsorgung ausgeschleust werden kann.

Durch die Verwendung der erfindungsgemäßen Membranfiltration wird, bedingt durch die hohe Recyclingrate, eine geringere hydraulische Abwasserfracht in die thermische Abwasserbehandlung eingebracht. Dadurch ergibt sich eine größenmäßige Reduktion bei der Auslegung neuer Abwasseranlagen sowie eine Kapazitätserhöhung bei bestehenden Abwasseranlagen.

In einer bevorzugten Ausführungsform des Hochdruckverfahrens wird das traizinhaltige Wasser einer Ultrafiltration zugeführt, das Permeat der Ultrafiltration der Nanofltration zugeführt und das Retentat der Ultrafiltration wird gemeinsam mit dem Permeat der Nanofiltration in die Melaminanlage rückgeführt.

Auf diese Weise können Grobbestandteile des triazinhaltigen Wassers in der Ultrafiltration abgeschieden werden, was eine Schonung der Nanofiltrationsmembran gewährleistet.

Über den Druck der Ultrafiltration kann das Mengenverhältnis Retentat : Permeat eingestellt werden. Dieses Verhältnis bestimmt die Konzentration an ionischen Triazinen im rückgeführten triazinhaltigen Wasser und damit im triazinhaltigen Wasserkreislauf der Melaminanlage.

Mit dem erfindungsgemäßen Verfahren zur Behandlung von triazinhaltigem Wasser einer Melaminanlage werden mehrere Vorteile erzielt.
Durch die spezifische Trennung zwischen ionischen Triazinen, wie Melamin, und nichtionischen Triazinen in der Membranfiltrationseinheit kann ein Großteil des im triazinhaltigen Wasser enthaltenen Melamins in den Prozess rückgeführt werden. Dadurch wird über den gesamten Melaminprozess die Ausbeute erhöht. Des weiteren ist die Konzentration an unerwünschten ionischen Triazinen im rückgeführten Strom erniedrigt. Eine niedrige Konzentration an ionischen Triazinen im triazinhaltigen Wasserkreislauf der Melaminanlage ist erwünscht, da auf diese Weise der Frischwasserbedarf im Aufarbeitungsteil der Melaminanlage reduziert wird. Darüber hinaus kann das Verfahren in flüssiger Phase und kontinuierlich durchgeführt werden.

Die Erfindung wird anhand von Ausführungsbeispielen in Zeichnungen dargestellt. Es zeigen:
- Fig. 1: einen Kreislauf des triazinhaltigen Wassers in einer Melaminanlage, welche keine Membranfiltrationseinheit aufweist (Stand der Technik);
- Fig. 2: einen Kreislauf des triazinhaltigen Wassers in einer Melaminanlage, welche eine Nanofiltration mit vorgeschalteter Ultrafiltration aufweist.

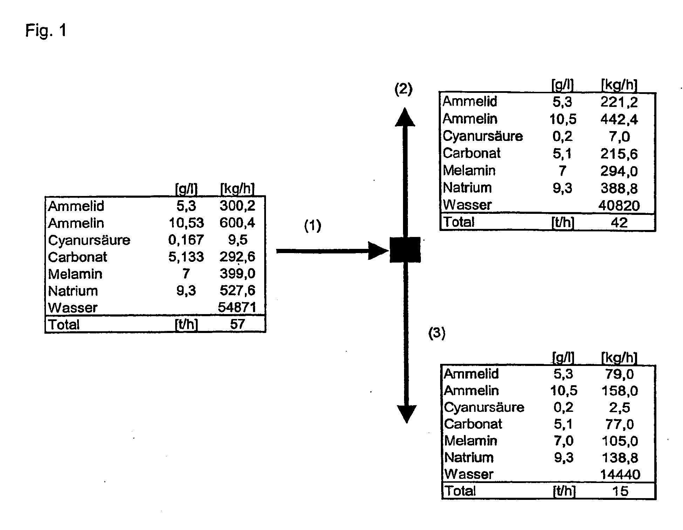

In Fig. 1 wird ein Fließschema ohne das erfindungsgemäße Verfahren dargestellt, bei dem die Auftrennung eines triazinhaltigen Wassers 1 dargestellt wird. Der trizinhaltige Wasserstrom 1 stammt aus einem hier nicht dargestellten Melaminanlage. Von anfallenden 57 t/h an triazinhaltigen Wasser 1 werden ohne Behandlung in einer Membranfiltrationseinheit 42 t/h, entsprechend 73 % der gesamten triazinhaltigen Wassermenge, in den wässrigen Aufarbeitungsteil der Melaminanlage rückgeführt 2. Die restlichen 15 t/h, entsprechend 27 % der triazinhaltigen Wassermenge werden in eine Abwasseranlage ausgeschleust 3. Dort werden die Inhaltsstoffe durch thermische Hydrolyse zersetzt und das erhaltene gereinigte Wasser anschließend entsorgt.

Da die beiden triazinhaltigen Wasserströme, wie aus Fig. 1 ersichtlich, dieselbe Konzentration an Inhaltsstoffen aufweisen, geht mit der Ausschleusung in die Abwasseranlage ein relativ großer Melaminverlust, nämlich 105 kg/h einher. Andererseits ist die über den rückgeführten triazinhaltigen Wasserstrom gemeinsam mit dem Melamin wieder in den Prozess eingetragene Nebenproduktmenge mit insgesamt 886,2 kg/h Ammelid, Ammelin, Cyanursäure und Carbonat beträchtlich.

Diese Menge an rezyklierten Nebenprodukten limitiert die in einem Prozess gemäß Fig. 1 maximal rückführbare triazinhaltige Wassermenge.

Im wässrigen Aufarbeitungsteil der Melaminanlage werden aus der vom Hochdruckteil kommenden Melaminschmelze alle Nebenprodukte abgetrennt. Dies geschieht dadurch, dass das Melamin selektiv auskristallisiert wird, während die Nebenprodukte quantitativ in der Kristallisationsmutterlauge gelöst werden. Für die quantitative Abtrennung ist es erforderlich, dass die Sättigungskonzentration an Nebenprodukten in der triazinhaltigen Mutterlauge nicht überschritten wird, andernfalls wäre das auskristallisierte Melamin mit Nebenprodukten verunreinigt.

Da der in den wässrigen Aufarbeitungsteil rezyklierte triazinhaltige Wasserstrom bereits an Nebenprodukten gesättigt ist, muss dem Prozess eine ausreichende Menge an Frischwasser zugeführt werden, um die quantitative Lösung der zusätzlichen Nebenprodukte aus der Melaminschmelze zu gewährleisten.

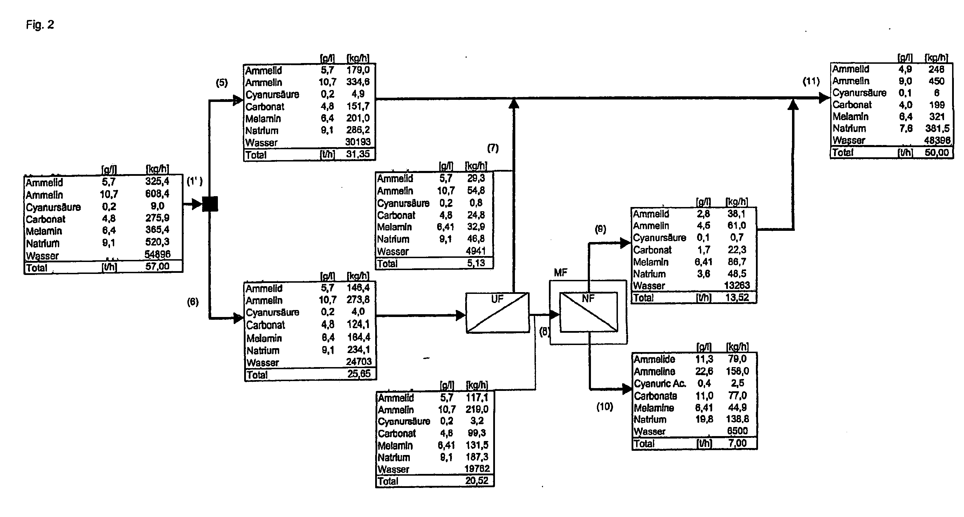

In einer Ausführungsform der Erfindung, die in Fig. 2 dargestellt ist, fallen im Melaminprozess 57 t/h eines triazinhaltigen Wassers 1' an, welches nahezu dieselbe Konzentration an Inhaltsstoffen aufweist wie diejenige aus dem in Fig. 1 beschriebenen Prozess.

31,4 t/h dieses triazinhaltigen Wassers, entsprechend 55 % der-Gesamtmenge, werden analog zum Verfahren gemäß Fig. 1 als Bypassstrom 5 ohne Behandlung in den Melaminprozess rückgeführt 25,7 t/h, entsprechend 45 % des triazinhaltigen Wassers 6, werden einer Ultrafiltrationseinheit UF zugeführt. In der Ultrafiltrationseinheit UF kommt es zum Abtrennen von Schwebstoffen und größeren Teilchen, welche im triazinhaltigen Wasser vorhanden sind. Diese sind im Retentat 7 der Ultrafiltration enthalten und werden gemeinsam mit Strom 5 in den Prozess rückgeführt. Das Trennprinzip der Ultrafiltration beruht ausschließlich auf Partikelgröße, hinsichtlich der Konzentration der Inhaltsstoffe kommt es durch die Ultrafiltration zu keiner Änderung.

Das Permeat 8 der Ultrafiltration wird einer Membranfiltrationseinheit MF (in Fig. 2 gestrichelt dargestellt) zugeführt, die hier eine Nanofiltration NF aufweist. Die Nanofiltration NF ist wie die Umkehrosmose und die Ultrafiltration ein druckgetriebenes Membranverfahren zur Trennung von gelösten Komponenten, insbesondere aus wässrigen Lösungen. Die Nanofiltration ist hinsichtlich ihres Trennverhaltens zwischen Umkehrosmose und Ultrafiltration einzuordnen. Eine Besonderheit von Nanofiltrationsmembranen ist ihre hohe lonenslektivität. Salze mit einwertigen Ionen passieren die Membran in hohem Maße. Salze mit mehrwertigen Ionen werden in höherem Maße zurückgehalten.

Die Membranfiltrationseinheit MF dient der Auftrennung in eine Fraktion, die an ionischen Triazinen reich ist und eine Fraktion, die an nichtionischen Triazinen reich ist.

Als Membran wird in der Nanofiltration NF eine Compositmembran verwendet, die Temperatur betrug 60 °C, der Druck bei der Nanofiltration betrug 20 bar. Es werden 13,5 t/h Permeat 9 und 7 t/h Retentat 10 erhalten. Während das Permeat 9 gemeinsam mit dem Retentat 10 der Ultrafiltration 7 und dem Bypassstrom 5 in die Melaminanlage rückgeführt wird, wird das Retentat 10 der Nanofiltration NF in die Abwasseranlage ausgeschleust.

Das Mengenverhältnis Permeat / Retentat der Nanofiltration beträgt 1,9 / 1. Während die Konzentration an nichtionischem Melamin nach der Nanofiltration NF unverändert ist, weist das Permeat 9 eine geringere Konzentration an ionischem Ammelid, Ammelin, Cyanursäure und Carbonat auf als im Zulaufstrom 8. Das Retentat 10 weist gegenüber dem Zulauf eine entsprechend höhere Konzentration an ionischen Verbindungen auf.

Da die Konzentration an Nebenprodukten im ausgeschleusten Retentat 10 bei gleicher Absolutmenge an abzuführenden Nebenprodukten höher als im Vergleichsprozess aus Fig. 1 ist, ist die hydraulische Abwassermenge mit 7 t/h um mehr als die Hälfte geringer als im Vergleichsprozess. Dies bedeutet eine geringere Belastung der Abwasseranlage, deren Kapazität dadurch steigt. Durch die verringerte Nebenproduktkonzentration im rezyklierten Permeat 9 kann eine größere Menge an triazinhaltigem Wasser in den Melaminprozess rückgeführt 2' werden, es sind dies 50 t/h im Vergleich zu 42 t/h beim Vergleichsprozess. Auf dieser Weise wird eine Einsparung von Frischwasser in der Melaminanlage erreicht. Aufgrund der höheren Melaminkonzentration im rückgeführten Strom 2' können 321 kg/h Melamin rückgewonnen werden, während im Vergleichsprozess 294 kg/h rezykliert werden.

Dies bedeutet für das erfindungsgemäße Verfahren eine Steigerung der Melaminausbeute.

### Bezugszeichenliste

- 1: triazinhaltiges Wasser aus einem Melaminprozess in Fig. 1
- 1': triazinhaltiges Wasser aus einem Melaminprozess in Fig. 2
- 2: in den Melaminprozess rückgeführtes triazinhaltiges Wasser in Fig. 1
- 2': in den Melaminprozess rückgeführtes triazinhaltiges Wasser in Fig. 2
- 3: in die Abwasseranlage ausgeschleustes triazinhaltiges Wasser

- 5: Bypassstrom zur Membranfiltration
- 6: der Ultrafiltration zugeführtes triazinhaltiges Wasser
- 7: Retentat der Ultrafiltration
- 8: Permeat der Ultrafiltration
- 9: Permeat der Nanofiltration
- 10: Retentat der Nanofiltration

- MF: Membranfiltrationseinheit
- UF: Ultrafiltration
- NF: Nanofiltration

## Patentansprüche

1. Verfahren zur Behandlung von triazinhaltigem Wasser einer Melaminanlage,
**dadurch gekennzeichnet, dass**
- das ionische und nichtionische Triazine in gelöster form enthaltende Wasser mindestens einer Membranfiltrationseinheit (MF) zugeführt wird,
- das Wasser in der Membranfiltrationseinheit (MF) in eine an ionischen Triazinen reiche und eine an nichtionischen Triazinen reiche Fraktion aufgetrennt wird,
- worauf die an ionischen Triazinen reiche Fraktion (10) ausgeschleust und die an nichtionischen Triazinen reiche Fraktion (9) in die Melaminanlage rückgeführt wird.

2. Verfahren nach Anspruch 1, **dadurch gekennzeichnet, dass** nach der Membranfiltrationseinheit (MF) die an ionischen Triazinen reiche Fraktion im Retentat (10) und die an nichtionischen Triazinen reiche Fraktion im Permeat (9) enthalten ist.

3. Verfahren nach Anspruch 1 oder 2, **dadurch gekennzeichnet, dass** die an ionischen Triazinen reiche Fraktion Natriumsalze des Ammelins und Ammelids enthält.

4. Verfahren nach mindestens einem der vorher genannten Ansprüche, **dadurch gekennzeichnet, dass** die an nichtionischen Triazinen reiche Fraktion Melamin enthält.

5. Verfahren nach mindestens einem der vorher genannten Ansprüche, **dadurch gekennzeichnet, dass** die Membranfiltrationseinheit (MF) eine oder mehrere Filtrationsstufen enthält.

6. Verfahren nach mindestens einem der vorher genannten Ansprüche, **dadurch gekennzeichnet, dass** die Membranfiltrationseinheit (MF) eine Nanofiltration (NF) und / oder Umkehrosmose ist.

7. Verfahren nach mindestens einem der vorher genannten Ansprüche, **dadurch gekennzeichnet, dass** die Membran eine aus mehreren Materialschichten aufgebaute Compositmembran ist.

8. Verfahren nach mindestens einem der vorher genannten Ansprüche, **dadurch gekennzeichnet, dass** die Membran eine gewickelte Fläche ist.

9. Verfahren nach mindestens einem der vorher genannten Ansprüche, **dadurch gekennzeichnet, dass** als Membranmaterial Polysulfone wie beispielsweise sulfonierte Polysulfone oder Polyethersulfone dienen.

10. Verfahren nach mindestens einem der vorher genannten Ansprüche, **dadurch gekennzeichnet, dass** mindestens einer Membranfiltrationseinheit (MF) eine Ultrafiltration (UF) vorgeschaltet ist und das Permeat der Ultrafiltration (8) der Membranfiltrationseinheit (MF) zugeführt wird.

11. Verfahren nach mindestens einem der vorher genannten Ansprüche, **dadurch gekennzeichnet, dass** das triazinhaltige Wasser aus dem Melaminprozess (1') einer Ultrafiltration (UF) zugeführt wird, das Permeat der Ultrafiltration (8) einer Nanofiltration (NF) und das Permeat der Nanofiltration (9) einer Umkehrosmose zugeführt wird.

12. Verfahren nach mindestens einem der vorher genannten Ansprüche, **dadurch gekennzeichnet, dass** das triazinhaltige Wasser Melaminkristallisationsmutterlauge enthält.

13. Verfahren nach mindestens einem der vorher genannten Ansprüche, **dadurch gekennzeichnet, dass** das triazinhaltige Wasser einen pH Wert von 11 bis 13 aufweist.

14. Verfahren nach mindestens einem der vorher genannten Ansprüche, **dadurch gekennzeichnet, dass** die Membranfiltrationseinheit (MF) bei einer Temperatur von 20 bis 95 °C betrieben wird.

15. Verfahren nach Anspruch 13, **dadurch gekennzeichnet, dass** die Membranfiltrationseinheit (MF) bei einer Temperatur von 50 bis 70 °C betrieben wird.

16. Verfahren nach mindestens einem der vorher genannten Ansprüche, **dadurch gekennzeichnet, dass** die Membranfiltrationseinheit (MF) bei einem Druck von 0,1 bis 100 bar betrieben wird.

17. Verfahren nach Anspruch 14, **dadurch gekennzeichnet, dass** die Membranfiltrationseinheit (MF) bei einem Druck von 10 bis 30 bar betrieben wird.

18. Verfahren nach mindestens einem der vorher genannten Ansprüche, **dadurch gekennzeichnet, dass** nach der Membranfiltrationseinheit (MF) das Konzentrationsverhältnis der nichtionischen Triazine im Permeat (9) : Retentat (10) 0,7 bis 1,1 beträgt.

19. Verfahren nach einem der vorher genannten Ansprüche, **dadurch gekennzeichnet, dass** nach der Membranfiltrationseinheit (MF) das Konzentrationsverhältnis der ionischen Komponenten im Permeat (9) : Retentat (10) 0,0 bis 0,9 beträgt.

20. Verfahren nach mindestens einem der vorher genannten Ansprüche, **dadurch gekennzeichnet, dass** nach der Membranfiltrationseinfieit (MF) das Massenverhältnis von Permeat (9) : Retentat (10) 1,5 beträgt.

21. Verfahren nach Anspruch 18, **dadurch gekennzeichnet, dass** nach der Membranfiltrationseinheit (MF) das Massenverhältnis von Permeat (9) : Retentat (10) 1,5 bis 20 beträgt.

22. Hochdruckverfahren zur Herstellung von Melamin aus Harnstoff, **dadurch gekennzeichnet, dass** das triazinhaltige Wasser aus einem Melaminprozess (1') mindestens einer Membranfiltrationseinheit (MF) zugeführt wird und **dadurch** in eine an ionischen Triazinen reiche (10) und eine an nichtionischen Triazinen reiche Fraktion (9) aufgetrennt wird, worauf die an ionischen Triazinen reiche Fraktion (10) in eine Anlage zur thermischen Abwasserbehandlung bei > 130 °C ausgeschleust und die an nichtionischen Triazinen reiche Fraktion (9) in den Nassteil der Melaminanlage rückgeführt wird.

23. Hochdruckverfahren nach Anspruch 21, **dadurch gekennzeichnet, dass** das triazinhaltige Wasser (1') einer Ultrafiltration (UF) zugeführt wird, das Permeat der Ultrafiltration (8) der Nanofiltration (NF) zugeführt wird und das Retentat der Ultrafiltration (7) gemeinsam mit dem Permeat der Nanofiltration (9) in die Melaminanlage rückgeführt wird.

## Claims

1. A process for treating triazine-containing water of a melamine plant
**characterized in that**
- the water comprising ionic and nonionic triazines in dissolved form is fed to at least one membrane filtration unit (MF),
- the water is separated in the membrane filtration unit (MF) into a fraction rich in ionic triazines and a fraction rich in nonionic triazines,
- then the fraction (10) rich in ionic triazines is discharged and the fraction (9) rich in nonionic triazines is recycled into the melamine plant.

2. The process as claimed in claim 1, **characterized in that**, downstream of the membrane filtration unit (MF), the fraction rich in ionic triazines is present in the retentate (10) and the fraction rich in nonionic triazines in the permeate (9).

3. The process as claimed in claim 1 or 2, **characterized in that** the fraction rich in ionic triazines comprises sodium salts of ammeline and ammelide.

4. The process as claimed in at least one of the preceding claims, **characterized in that** the fraction rich in nonionic triazines comprises melamine.

5. The process as claimed in at least one of the preceding claims, **characterized in that** the membrane filtration unit (MF) comprises one or more filtration stages.

6. The process as claimed in at least one of the preceding claims, **characterized in that** the membrane filtration unit (MF) is a nanofiltration (NF) and/or reverse osmosis.

7. The process as claimed in at least one of the preceding claims, **characterized in that** the membrane is a composite membrane formed from a plurality of material layers.

8. The process as claimed in at least one of the preceding claims, **characterized in that** the membrane is a wound surface.

9. The process as claimed in at least one of the preceding claims, **characterized in that** the membrane materials used are polysulfones, for example sulfonated polysulfones or polyether sulfones.

10. The process as claimed in at least one of the preceding claims, **characterized in that** an ultrafiltration (UF) is connected upstream of at least one membrane filtration unit (MF) and the permeate of the ultrafiltration (8) is fed to the membrane filtration unit (MF).

11. The process as claimed in at least one of the preceding claims, **characterized in that** the triazine-containing water from the melamine process (1') is fed to an ultrafiltration (UF), the permeate of the ultrafiltration (8) is fed to a nanofiltration (NF) and the permeate of the nanofiltration (9) is fed to a reverse osmosis.

12. The process as claimed in at least one of the preceding claims, **characterized in that** the triazine-containing water comprises melamine crystallization mother liquor.

13. The process as claimed in at least one of the preceding claims, **characterized in that** the triazine-containing water has a pH of from 11 to 13.

14. The process as claimed in at least one of the preceding claims, **characterized in that** the membrane filtration unit (MF) is operated at a temperature of from 20 to 95°C.

15. The process as claimed in claim 13, **characterized in that** the membrane filtration unit (MF) is operated at a temperature of from 50 to 70°C.

16. The process as claimed in at least one of the preceding claims, **characterized in that** the membrane filtration unit (MF) is operated at a pressure of from 0.1 to 100 bar.

17. The process as claimed in claim 14, **characterized in that** the membrane filtration unit (MF) is operated at a pressure of from 10 to 30 bar.

18. The process as claimed in at least one of the preceding claims, **characterized in that**, downstream of the membrane filtration unit (MF), the concentration ratio of the nonionic triazines in the permeate (9) : retentate (10) is from 0.7 to 1.1.

19. The process as claimed in one of the preceding claims, **characterized in that**, downstream of the membrane filtration unit (MF), the concentration ratio of the ionic components in the permeate (9) : retentate (10) is from 0.0 to 0.9.

20. The process as claimed in at least one of the preceding claims, **characterized in that**, downstream of the membrane filtration unit (MF), the mass ratio of permeate (9) : retentate (10) is 1.5.

21. The process as claimed in claim 18, **characterized in that**, downstream of the membrane filtration unit (MF), the mass ratio of permeate (9) : retentate (10) is from 1.5 to 20.

22. A high-pressure process for preparing melamine from urea, **characterized in that** the triazine-containing water from a melamine process (1') is fed to at least one membrane filtration unit (MF) and separated thereby into a fraction (10) rich in ionic triazines and a fraction (9) rich in nonionic triazines, then the fraction (10) rich in ionic triazines is discharged into a plant for thermal wastewater treatment at > 130°C, and the fraction (9) rich in nonionic triazines is recycled into the wet part of the melamine plant.

23. The high-pressure process as claimed in claim 21, **characterized in that** the triazine-containing water (1') is fed to an ultrafiltration (UF), the permeate of the ultrafiltration (8) is fed to the nanofiltration (NF), and the retentate of the ultrafiltration (7) is recycled together with the permeate of the nanofiltration (9) into the melamine plant.

## Revendications

1. Procédé pour le traitement d'eau contenant de la triazine d'une installation de mélamine, **caractérisé**
- **en ce que** de l'eau contenant de la triazine ionique et non ionique sous forme dissoute est introduite dans au moins une unité de filtration à membrane (MF),
- **en ce que** l'eau est séparée dans l'unité de filtration à membrane (MF) en une fraction riche en triazines ioniques et en une fraction riche en triazines non ioniques,
- suite à quoi la fraction riche en triazines ioniques (10) est évacuée et la fraction riche en triazines non ioniques (9) est recyclée dans l'installation de mélamine.

2. Procédé selon la revendication 1, **caractérisé en ce qu'**après l'unité de filtration à membrane (MF) la fraction riche en triazines ioniques (10) est contenue dans le rétentat et la fraction riche en triazines (9) non ioniques est contenue dans le perméat.

3. Procédé selon la revendication 1 ou 2, **caractérisé en ce que** la fraction riche en triazines ioniques contient des sels de sodium de l'ammeline et de l'ammelide.

4. Procédé selon au moins l'une quelconque des revendications susmentionnées, **caractérisé en ce que** la fraction riche en triazines non ioniques contient de la mélamine.

5. Procédé selon au moins l'une quelconque des revendications susmentionnées, **caractérisé en ce que** l'unité de filtration à membrane (MF) contient une ou plusieurs étapes de filtration.

6. Procédé selon au moins l'une quelconque des revendications susmentionnées, **caractérisé en ce que** l'unité de filtration à membrane (MF) est une nanofiltration (NF) et/ou une osmose inverse.

7. Procédé selon au moins l'une quelconque des revendications susmentionnées, **caractérisé en ce que** la membrane est une membrane composite formée par plusieurs couches de matériaux.

8. Procédé selon au moins l'une quelconque des revendications susmentionnées, **caractérisé en ce que** la membrane est une surface enroulée.

9. Procédé selon au moins l'une quelconque des revendications susmentionnées, **caractérisé en ce qu'**on utilise comme matériau de membrane des polysulfones telles que par exemple des polysulfones sulfonées ou des polyéthersulfones.

10. Procédé selon au moins l'une quelconque des revendications susmentionnées, **caractérisé en ce qu'**au moins une unité de filtration à membrane (MF) est précédée d'une ultrafiltration (UF) et le perméat de l'ultrafiltration (8) est introduit dans l'unité de filtration à membrane (MF).

11. Procédé selon au moins l'une quelconque des revendications susmentionnées, **caractérisé en ce que** l'eau contenant de la triazine provenant du processus de mélamine (1') est introduite dans une ultrafiltration (UF), le perméat de l'ultrafiltration (8) est introduit dans une nanofiltration (NF) et le perméat de la nanofiltration (9) est introduit dans une osmose inverse.

12. Procédé selon au moins l'une quelconque des revendications susmentionnées, **caractérisé en ce que** l'eau contenant de la triazine contient de la lessive-mère de la cristallisation de la mélamine.

13. Procédé selon au moins l'une quelconque des revendications susmentionnées, **caractérisé en ce que** l'eau contenant de la triazine présente un pH de 11 à 13.

14. Procédé selon au moins l'une quelconque des revendications susmentionnées, **caractérisé en ce que** l'unité de filtration à membrane (MF) est opérée à une température de 20 à 95°C.

15. Procédé selon la revendication 13, **caractérisé en ce que** l'unité de filtration à membrane (MF) est opérée à une température de 50 à 70°C.

16. Procédé selon au moins l'une quelconque des revendications susmentionnées, **caractérisé en ce que** l'unité de filtration à membrane (MF) est opérée à une pression de 0,1 à 100 bars.

17. Procédé selon la revendication 14, **caractérisé en ce que** l'unité de filtration à membrane (MF) est opérée à une pression de 10 à 30 bars.

18. Procédé selon au moins l'une quelconque des revendications susmentionnées, **caractérisé en ce qu'**après l'unité de filtration à membrane (MF) le rapport des concentrations de la triazine non ionique dans le perméat (9):rétentat (10) est de 0,7 à 1,1.

19. Procédé selon l'une quelconque des revendications susmentionnées, **caractérisé en ce qu'**après l'unité de filtration à membrane (MF) le rapport des concentrations des composants ioniques dans le perméat (9):rétentat (10) est de 0,0 à 0,9.

20. Procédé selon au moins l'une quelconque des revendications susmentionnées, **caractérisé en ce qu'**après l'unité de filtration à membrane (MF) le rapport massique de perméat (9):rétentat (10) est de 1,5.

21. Procédé selon la revendication 18, **caractérisé en ce qu'**après l'unité de filtration à membrane (MF) le rapport massique de perméat (9):rétentat (10) est de 1,5 à 20.

22. Procédé sous haute pression de préparation de mélamine à partir d'urée, **caractérisé en ce que** l'eau contenant la triazine provenant d'un procédé de mélamine (1') est introduite dans au moins une unité de filtration à membrane (MF) et est ainsi séparée en une fraction riche en triazines ioniques (10) et en une fraction riche en triazines non ioniques (9), suite à quoi la fraction riche en triazines ioniques (10) est évacuée dans une installation pour le traitement thermique des eaux usées à > 130°C et la fraction riche en triazines non ioniques (9) est recyclée dans la partie humide de l'installation de mélamine.

23. Procédé sous haute pression selon la revendication 21, **caractérisé en ce que** l'eau contenant de la triazine (1') est introduite dans une ultrafiltration (UF), le perméat de l'ultrafiltration (8) est introduit dans la nanofiltration (NF) et le rétentat de l'ultrafiltration (7) est recyclé ensemble avec le perméat de la nanofiltration (9) dans l'installation de mélamine.
